# EUROPEAN PATENT SPECIFICATION

(11) **EP 0 872 246 B1**
(45) Date of publication and mention of the grant of the patent: **19.02.2003**
(21) Application number: 96939325.5
(22) Date of filing: 29.11.1996
(51) Int. Cl.: A61K 45/00, A61K 31/135, A61K 31/415, A61K 31/47, A61K 31/167, A61K 31/4704, A61K 31/4184, A61P 13/04

(54) **DRUG FOR RELIEVING PAIN AND PROMOTING THE REMOVAL OF CALCULI IN UROLITHIASIS**
MEDIKAMENT ZUR LINDERUNG VON SCHMERZEN UND ZUR FÖRDERUNG DER ENTFERNUNG VON KONKREMENTEN BEI UROLITHIASIS
MEDICAMENT POUR ATTENUER LA DOULEUR ET FAVORISER L'ELIMINATION DES CALCULS LIES A L'UROLITHIASE

(30) Priority: 30.11.1995 JP 34659995
(43) Date of publication of application: 21.10.1998
(73) Proprietor: KISSEI PHARMACEUTICAL CO., LTD., Matsumoto-City Nagano-Pref. 399 (JP)
(72) Inventor: AKAHANE, Masuo, Matsumoto-shi, Nagano 390-02 (JP); TOMIYAMA, Yoshitaka, Matsumoto-shi, Nagano 399 (JP)
(74) Representative: Baverstock, Michael George Douglas
(86) International application number: PCT/JP96/03494
(87) International publication number: WO 97/019700

(56) References cited:
- EP-A- 0 252 286
- LONGRIGG J.N.: "the renal calyces, pelvis and ureter. experimental and clinical studies " J. R. COLL. SURG. EDINBURGH, vol. 22, no. 5, 1977, pages 309-318, XP002085648
- LINDSEY ET AL: "modification by dipyrone of stone-induced ureteric hyperperistalsis in the dog" UROL. RES., vol. 7, no. 1, 1979, pages 13-17, XP002085649
- BLOCH ET AL: "effects of theophylline and isoproterenol on the activity on the isolated guinea-pig ureter" UROL. INT., vol. 39, no. 5, 1984, pages 308-311, XP002085650
- UROLOGE, Jul. 1971, Vol. 10, No. 4, MELCHIOR H., "Spasmolyse Durch Beta-Adrenergica. Ein Beitrag Zur Konservativen Behandlung Der Urolithiasis", pages 183-188.
- UROLOGIA INTERNATIONALIS, 1990, Vol. 45, No. 1, MORITA T., "Cyclic Adenosine Monophosphate Production and Contractile Response Induced by beta-Adrenoceptor Subtypes in Rabbit Urinary Bladder Smooth Muscle", pages 10-15.
- Robinson E., Hudson A.: Adrenoceptor Pharmacology, pages 1/6-6/6

## Description

### Technical Field

The present invention relates to the use of a drug having selective stimulating effects on both β₂- and β₃-adrenoceptors as an active ingredient in the manufacture of a pharmaceutical composition for relieving pain related to urolithiasis and promoting the removal of calculi in urolithiasis.

### Background Art

It is known that there are three subtypes of β-adrenoceptor, which have been classified as β₁, β₂ and β₃. Each receptor subtype is distributed in specified organs. β₁-Adrenoceptor is mainly present in the heart and its stimulation enhances the function of the heart. β₂-Adrenoceptor is mainly present in the trachea, peripheral blood vessels and the uterus, and smooth muscle of these organs is relaxed by the stimulation of this receptor. In addition, it has been recently reported that β₃-adrenoceptor is present in the digestive tract and adipocytes. The stimulation of β₃-adrenoceptor leads to the relaxation of gastrointestinal smooth muscle, lipolysis and energy expenditure in adypose tissues and so on.

Thus, the distribution of β-adrenoceptor subtypes is specified by organs and tissues. Various receptor subtypes including β-adrenoceptor have been actively studied with a view to developing more effective medicinal treatment of some diseases. Consequently, efforts have been made extensively to develop more effective and highly selective drugs that act on specified organs. However, β-adrenoceptor subtypes distributed in the human ureter have not been elucidated at all, and the progress of studies to develop drug that act more effectively on the human ureter has been desired.

Urolithiasis is a disease generating calculi in the lumen of the entire urinary tract from the kidney to the urethra. The calculi are thought to be formed in a series of events such as nucleation of the urinary component, crystallization, aggregation, concretion and enlargement. Urinary flow is often obstructed by calculi, which results in the rise of intra-ureteral pressure, leading to pain. An analgesic and an antispastic are prescribed for the pain. However, the use of the analgesic is only a temporary symptomatic therapy for the pain, and is not expected to treat urolithiasis fundamentally at all. The effectiveness of an anti-cholinergic, one of the antispastics, is also not satisfactory. Therefore, drugs which relieve pain and promote the removal of calculi by widening the ureter with their strong relaxing effects are desired (The Journal of Urology, Vol.152, pp.1095-1098 (1994)).

UROLOGE.Jul.1971.Vol.10,No.4, pages 183-188, discloses that β-adrenergic agonists such as isoproterenol and orciprenalin have a spasmolytic effect on the ureter based upon the inhibition of human ureteral peristalsis.

### Disclosure of Invention

The inventors have studied extensively drug effects on the human ureter to elucidate the β-adrenoceptor subtypes distributed in the human ureter. As a result, it was found surprisingly that β₃-adrenoceptor in addition to β₂-adrenoceptor are present in the human ureter, thereby accomplishing the present invention.

The present invention provides the use of a drug having selective stimulating effects on both β₂- and β₃-adrenoceptors as active ingredient in the manufacture of a pharmaceutical composition for relieving pain related to urolithiasis and promoting the removal of calculi in urolithiasis.

The novel drug for relieving pain and promoting the removal of calculi in urolithiasis contains as active ingredient a drug which exerts a strong relaxing effect on human ureteral smooth muscle by stimulating both β₂- and β₃-adrenoceptors.

### Brief Description of Drawings

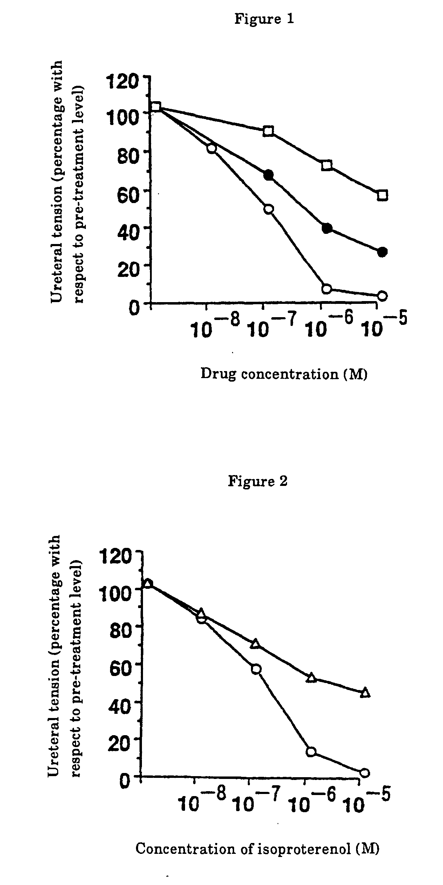

Figure 1 illustrates the effect of each drug on the contraction of isolated human ureteral smooth muscle induced by 40 mM of potassium chloride. The axis of the ordinates shows the change of ureteral tension after treatment by each drug. The ureteral tension before the treatment is indicated as 100 %, and the tension after the treatment by 10⁻⁵ M of isoproterenol which produces maximal relaxation of the ureter is indicated as 0 %. The axis of the abscissas shows drug concentrations (M). The symbols -O-, -●- and -□- show isoproterenol, procaterol and CGP-12,177A, respectively.

Figure 2 illustrates the effect of each drug on the contraction of isolated human ureteral smooth muscle induced by 40 mM of potassium chloride. The axis of ordinates shows the change of ureteral tension after treatment with each drug. The ureteral tension before the treatment is indicated as 100 %, and the tension after the treatment with 10⁻⁵ M of isoproterenol alone which produces maximal relaxation of the ureter is indicated as 0 %. The axis of the abscissas shows concentrations (M) of isoproterenol. The symbols - O- and -Δ- show isoproterenol alone and isoproterenol in the presence of 100 nM of ICI-118,551, respectively.

### Best Mode for Carrying Out the Invention

The present inventors carried out the following experiments using isoproterenol as a nonselective β-adrenoceptor stimulant, procaterol as a selective β₂-adrenoceptor stimulant, CGP-12,177A hydrochloride [chemical name: (±)-4-[3-[(1,1-dimethylethyl)amino]-2-hydroxypropoxy]-1,3-dihydro-2H-benzimidazol-2-one hydrochloride] as a selective β₃-adrenoceptor stimulant with β₁- and β₂-adrenoceptors blocking activities (Molecular Pharmacology, Vol.44, pp.1094-1104 (1993)), and ICI-118,551 hydrochloride [chemical name: (±)-1-[(2,3-dihydro-7-methyl-1H-inden-4-yl)oxy]-3-[(1-methylethyl)amino]-2-butanol hydrochloride] as a selective β₂-adrenoceptor blocker.

The effects of the drugs on the contraction of human ureteral smooth muscle induced by potassium chloride were investigated. It was found that both procaterol, a selective β₂-adrenoceptor stimulant, and CGP-12,177A, a selective β₃-adrenoceptor stimulant, produced apparent relaxation of the smooth muscle. This result indicates that β₃-adrenoceptor is present in the human ureter in addition to β₂-adrenoceptor.

Furthermore, the effects of isoproterenol alone and isoproterenol combined with ICI-118,551 were compared in a similar experiment using human ureteral smooth muscle. It was found that the relaxing effect of isoproterenol on ureteral smooth muscle is not blocked completely by ICI-118,551, a selective β₂-adrenoceptor blocker. Therefore, it was confirmed pharmacologically that β₃-adrenoceptor in addition to β₂-adrenoceptor is present in the human ureter.

Thus, it became clear that both β₂- and β₃-adrenoceptors are present in the human ureter. Specifically, drugs having selective stimulating effects on both β₂- and β₃-adrenoceptors can provide clinically satisfactory relaxation of the human ureter, which cannot be attained using the drugs hitherto used. Drugs having selective stimulating effects on both β₂- and β₃-adrenoceptors are very useful for relieving pain and promoting the removal of calculi in urolithiasis.

The drugs having selective stimulating effects on both β₂- and β₃-adrenoceptors of the present invention have excellent relaxing effects on human ureteral smooth muscle, and are useful for relieving pain, promoting spontaneous passage of calculi and the removal of calculi after extracorporeal shock wave lithotripsy and so on.

As drugs having selective stimulating effects on both β₂- and β₃-adrenoceptors, drugs exhibiting both β₂- and β₃-adrenoceptor stimulating effects simultaneously can be used in a range of usual dose. For example, formoterol (chemical name: 2-hydroxy-5-[(1RS)-1-hydroxy-2-[[(1RS)-2-(p-methoxyphenyl)-1-methylethyl]amino]ethyl]formanilide) can be illustrated.

In the EC₅₀ ratio of β₂-adrenoceptor stimulating effect to β₃-adrenoceptor stimulating effect, values ranging from about 1/100 to 100/1 are preferable, and those ranging from about 1/10 to 10/1 are more preferable.

Usually, EC₅₀ values for β₂-adrenoceptor stimulating effects can be calculated by measuring the inhibitory effects on the spontaneous uterine contractions of a pregnant rat. For example, the EC₅₀ value of formoterol was 4.2 x 10⁻¹⁰(M).

Usually, EC₅₀ values for β₃-adrenoceptor stimulating effects can be calculated by measuring the inhibitory effects on the spontaneous contractions of ferret ureteral smooth muscle. For example, the EC₅₀ value for formoterol was 5.1 x 10⁻⁸(M).

Drugs having markedly weakened β₁-adrenoceptor stimulating effects are preferred in order to reduce burdens on the heart and so as not to induce side effects such as tachycardia.

### Examples

The present invention is further illustrated in more detail by way of the following Examples.

### Measurement of the contractile force of ureteral smooth muscle strip

(1) Preparation of smooth muscle strips Specimens of human ureter were obtained from patients undergoing nephrectomy or total cystectomy. After the specimens were carefully dissected free from the surrounding fat tissues, they were cut into spiral strips. Ureteral strips of about 20 mm in length and 5 mm in width were prepared.
(2) Experimental conditions Buffer solution; Locke's-solution No.2: NaCl (154 mM), KCl (5.6 mM), CaCl₂ (2.1 mM), NaHCO₃ (5.6 mM) and glucose (3.6 mM)

Ureteral muscle strips without any spontaneous rhythmic contractions were used. An initial resting tension of about 1.0 g was set. The actions of drugs against the 40 mM KCl-induced tonic contraction were evaluated in the presence of 10⁻⁶ M phentolamine.

Conditions for measurement; The bathing solution was maintained at 37°C and gassed with a mixture of 95 % O₂ and 5 % CO₂.

Drug treatment; The drug solution was added cumulatively to the organ bath every 5 minutes.

Evaluation of the drugs; The drug-induced relaxation was expressed as a percentage of the maximal relaxing response to 10⁻⁵ M of isoproterenol. The ureteral tension before the treatment is indicated as 100 %, and the tension after treatment with 10⁻⁵ M of isoproterenol which produces maximal ureteral relaxation is indicated as 0 %.

### Example 1

According to the methods described above, the ureteral relaxing effects of the drug were measured using the human ureteral smooth muscle strips.

### Drugs: 1. isoproterenol, 2. procaterol and 3. CGP-12,177A hydrochloride

The results are as follows (Fig.1): CGP-12,177A, a selective β₃-adrenoceptor stimulant, as well as procaterol, a selective β₂-adrenoceptor stimulant, produced apparent ureteral relaxation. Moreover, isoproterenol, a non-selective β-adrenoceptor stimulant, which stimulates both β₂- and β₃-adrenoceptors, also showed a more potent relaxing effect. These results demonstrate that the relaxation of human ureteral smooth muscle is mediated via β₃₋ adrenoceptors in addition to β₂-adrenoceptors.

### Example 2

According to the method described above, the interaction between a β-adrenoceptor stimulant and a β-adrenoceptor blocker was evaluated in human ureteral smooth muscle strips using the following drugs.

### Drugs: 1. isoproterenol and 2. ICI-118,551 hydrochloride

The results are as follows (Fig. 2): The pre-treatment with ICI-118,551 (100nM), a selective β₂-adrenoceptor blocker, attenuated the isoproterenol-induced ureteral relaxation, but did not completely block it. Isoproterenol still produced a relaxation by about 50 %, even if 100 nM of ICI-118,551 was present. To judge from these results, it is also demonstrated that the relaxation of human ureteral smooth muscle is mediated via β₃-adrenoceptors in addition to β₂-adrenoceptors and that considerable numbers of β₃-adrenoceptors exist in human ureteral smooth muscle.

### Example 3

### Experiment for measuring the β₂-adrenoceptor stimulating effect

### (The effects of the drug on the spontaneous contraction in the isolated uterus of a pregnant rat).

The uterus of pregnant SD rats (21st day of pregnancy) was isolated and longitudinal smooth muscle strips (about 15 mm in length and 5 mm in width) free from the basal plate were prepared. The experiment was conducted according to the Magnus method. The preparations were mounted with an initial resting tension of about 1.0 g in the Locke's-Ringer solution maintained at 37°C and gassed with a mixture of 95 % O₂ and 5 % CO₂. The spontaneous contraction of the uterus was measured by an isometric force-transducer and recorded in a rectigraph. The drug solution was added cumulatively to the organ bath every 5 minutes. Uterine activities were calculated as the sum of the amplitudes of the spontaneous contraction for 5 minutes, and the percent change before and after the drug application were compared. The EC₅₀ value was determined as the molar concentration required to produce 50 % of the maximal relaxation elicited by each drug.

### Example 4

The experiment for measuring β₃-adrenoceptor stimulating effects.

### (The effects of the drug on the spontaneous rhythmic contraction in the isolated ferret ureter).

The ferret ureter was isolated and the ring smooth muscle strips were prepared (about 20 mm in length). The experiment was conducted according to the Magnus method. The preparations were mounted with an initial resting tension of about 0.5 g in the Krebs-Henseleit solution maintained at 37°C and gassed with a mixture of 95 % O₂ and 5 % CO₂. The spontaneous contraction of the ureter was measured by an isometric force-transducer and recorded in a rectigraph. The drug solution was added cumulatively to the organ bath every 5 minutes. Ureteral activities were calculated as the sum of the amplitudes of the spontaneous contraction for 5 minutes, and the percent change before and after the drug application were compared. The EC₅₀ value was determined as the molar concentration required to produce 50 % of the maximal relaxation elicited by each drug.

### Industrial Applicability

A pharmaceutical composition which contains as the active ingredient a drug having the selective stimulating effects on both β₂- and β₃-adrenoceptors of the present invention exerts a potent relaxing effect on human ureteral smooth muscle, and is suitable as a drug for relieving pain related to urolithiasis and promoting the removal of calculi in urolithiasis.

## Claims

1. Use of a drug having selective stimulating effects on both β₂- and β₃-adrenoceptors as an active ingredient in the manufacture of a pharmaceutical composition for relieving pain related to urolithiasis and promoting the removal of calculi in urolithiasis.

## Patentansprüche

1. Verwendung eines Wirkstoffes, der selektive stimulierende Wirkungen auf sowohl β₂- als auch β₃-Adrenozeptoren aufweist, als aktiven Bestandteil bei der Herstellung einer pharmazeutischen Zusammensetzung zur Linderung von Schmerzen, die mit Urolithiasis in Verbindung stehen, und zur Förderung der Entfernung von Calculi bei Urolithiasis.

## Revendications

1. Utilisation d'un médicament ayant des effets stimulateurs sélectifs à la fois sur les adrénorécepteurs β₂ et les adrénorécepteurs β₃ comme ingrédient actif dans la production d'une composition pharmaceutique pour soulager la douleur due à l'urolithiase et activer l'élimination des calculs dans l'urolithiase.
